# EUROPEAN PATENT APPLICATION

(11) **EP 4 796 196 A1**
(43) Date of publication of application: **26.08.2026**
(21) Application number: 25772259.5
(22) Date of filing: 17.03.2025
(51) Int. Cl.: B01D 3/42, B01D 5/00, H01M 10/04, C07D 207/267

(54) **NMP RECOVERY AND PURIFICATION SYSTEM AND CONTROL METHOD THEREFOR**

(30) Priority: 15.03.2024 KR 20240036253
(71) Applicant: LG Energy Solution, Ltd., Seoul 07335 (KR)
(72) Inventor: KIM, Geonyong, Daejeon 34122 (KR)
(74) Representative: Plasseraud IP
(86) International application number: PCT/KR2025/003413
(87) International publication number: WO 2025/193013

(57) **Abstract**

The present invention relates to an NMP purification system and a control method thereof, and more specifically, relates to an NMP recovery purification system and a control method thereof.

According to one example of the present invention, an NMP recovery purification system may be provided, which comprises a recovery device operating to recover NMP contained in exhaust gas discharged from a secondary battery process; a recovered NMP storage tank storing the recovered NMP recovered and discharged from the recovery device; a purification device operating to purify the recovered NMP supplied from the recovered NMP storage tank into high-purity NMP; a purified NMP storage tank storing the purified NMP purified and discharged from the purification device; and a circulation line circulating the purified NMP discharged from the purification device to the recovery device.

## Description

### Technical Field

The present invention relates to an NMP purification system and a control method thereof, and more specifically, relates to an NMP recovery purification system and a control method thereof.

This application claims the benefit of priority based on Korean Patent Application No. 10-2024-0036253 dated March 15, 2024, and Korean Patent Application No. 10-2025-0033834 dated March 17, 2025, the disclosures of which are incorporated herein by reference in their entirety.

### Background Art

When an electrode for a lithium-ion battery is manufactured, high-quality NMP (N-methyl-2-pidolidone) is used as a solvent.

In an electrode process for manufacturing an electrode, a coating process is performed in which a slurry is applied to an electrode base material and coated. That is, NMP is a component constituting the slurry, which is included in gas exhausted during a drying process of the coating process. NMP is particularly used in a mixing process of mixing a positive-electrode material.

Since NMP is very expensive and must be managed as a hazardous substance, the used NMP is recovered in a condensed state through a recovery device or recovery system for reuse.

The recovered NMP is purified through a purification device or purification system. The purified NMP can be reused in the coating process.

The purification device comprises a purification tower, where the purification device can be referred to as a device for producing high-purify purified NMP by purifying the recovered NMP. The purification device, especially the purification tower, performs purification in a state where the temperature and pressure are very precisely controlled. Therefore, it can take a long time from the operation of the purification tower to normal operation.

If the amount of purified NMP discharged from the purification tower is excessive, it is necessary to temporarily stop the operation of the purification tower. However, when the operation of the purification tower is stopped, the ratio of the gas and liquid phases, which are dynamic states inside the purification tower, changes rapidly, and it takes a long time to stabilize upon restarting, so that the purification yield is lowered, and moreover, the yield of the entire electrode production process can be affected.

Meanwhile, in the conventional NMP recovery system and NMP purification system, the operation stop of the purification tower is performed dependently by the operation stop of the recovery tower, and conversely, the operation stop of the recovery tower is performed dependently by the operation stop of the purification tower.

As one example, if the operation of the recovery tower is stopped in a state where the recovery tower and the purification tower are operated normally, the amount of recovered NMP flowing into the purification tower is insufficient, so that the operation of the purification tower is stopped. Even if the temporary operation of the recovery tower and the purification tower occurs, it takes a long time until the normal operation after restarting.

Therefore, it is necessary to find a method capable of effectively ensuring normal operation of the purification tower by effectively avoiding the operation stop or temporary stop of the purification tower. Furthermore, it is also necessary to find a method capable of effectively ensuring normal operation of the recovery tower for normal operation of the purification tower.

### Disclosure

### Technical Problem

Through one example of the present invention, it is intended to provide an NMP recovery purification system capable of maintaining normal operation of a purification tower despite a temporary stop condition of the purification tower by circulating NMP purified in the purification tower to a recovery tower, and a control method thereof.

Through one example of the present invention, it is intended to provide an NMP recovery purification system in which a circulation region is further expanded so that NMP purified in a purification tower is introduced into a recovery tower, and then introduced into the purification tower through the recovery tower again, instead of self-circulation in which it is introduced into the purification tower again, thereby capable of being in organic conjunction with the recovery tower and the purification tower, and securing safety and operational stability of the system, and a control method thereof.

Through one example of the present invention, it is intended to provide an NMP recovery purification system capable of effectively reducing an impurity content in NMP, which is re-introduced into a purification tower, by further diluting the already-generated NMP peroxide in a recovery tower, and a control method thereof.

Through one example of the present invention, it is intended to provide an NMP recovery purification system capable of effectively preventing operation stop of a purification tower due to operation stop of a recovery tower by enabling normal operation of the recovery tower even under an operation stop condition of the recovery tower, and a control method thereof.

### Technical Solution

In order to achieve the above-described object, according to one example of the present invention, an NMP recovery purification system may be provided, which comprises a recovery device operating to recover NMP contained in exhaust gas discharged from a secondary battery process; a recovered NMP storage tank storing the recovered NMP recovered and discharged from the recovery device; a purification device operating to purify the recovered NMP supplied from the recovered NMP storage tank into high-purity NMP; a purified NMP storage tank storing the purified NMP purified and discharged from the purification device; and a circulation line circulating the purified NMP discharged from the purification device to the recovery device.

The secondary battery process may be a coating process of coating a positive electrode material on an electrode base material, and NMP may be recovered and purified from exhaust gas discharged during a drying process of the coating process, and reused. Since the purity of the recovered NMP is relatively low, it cannot be used directly, and the recovered NMP may be purified to high purity and reused.

The recovery device may comprise a recovery tower, and the purification device may comprise a purification tower.

The recovery method of NMP may be very diverse, and thus, the recovery device may also be manufactured in various ways. The recovery tower may also be called an absorption tower, and a method in which NMP is absorbed into condensate therein and recovered may be performed. NMP in a liquid state may be recovered from the exhaust gas through the recovery tower. The recovery tower may be provided in a single tower or multiple towers, and the concentration of the recovered NMP may be increased through multiple recovery processes, and the content of impurities may be reduced.

Here, the impurity can be called NMP peroxide, and it cannot be overemphasized that the impurity should be removed as much as possible to ultimately converge to 100% purity.

The purification method of NMP can be very diverse, and therefore the recovery device can also be manufactured in various ways. Therefore, the purification device can also be manufactured in various ways. The purification tower can also be called a distillation tower, and the recovered NMP in a liquid state can be purified into high-purity NMP in a vapor state by heating it therein.

It is preferable that the purification tower has a top tower for separating high-concentration NMP and low-boiling-point impurities, a bottom tower for separating high-purity NMP and high-boiling-point impurities, and a middle tower for discharging high-purity purified NMP gas between the top tower and the bottom tower. That is, the inside of the purification tower may be provided with multiple layers, and functions performed at the respective locations may be distinguished.

In the upper part of the purification tower, low-boiling-point impurities such as water may be separated from high-concentration NMP. Therefore, high-purity NMP may be obtained through high-concentration NMP.

In the lower part of the purification tower, high-boiling point impurities such as NMP peroxide may be separated from high-purity NMP. Therefore, by removing impurities from high-purity NMP, higher purity NMP may be obtained.

In the middle part of the purification tower, high-purity NMP in a liquid state may be obtained in a gaseous state. Since a continuous distillation process is performed throughout the entire inside of the purification tower and the purified NMP in the middle part of the purification tower is discharged outside the purification tower, this can be called a side-cut distillation method.

The purification device may comprise a condenser for condensing the high-purity purified NMP gas discharged from the middle tower to supply it to the purified NMP storage tank. That is, the condenser is provided in a purified NMP line connecting the purification device and the purified NMP storage tank, and the high-purity purified NMP liquid condensed from gas to liquid is stored in the purified NMP storage tank.

It is preferable that the circulation line is provided to connect the purification tower and the recovery tower. Here, the circulation line is a pipe allowing a fluid flow to be performed from the purification tower to the recovery tower, and it is preferable that the fluid flow in the opposite direction is blocked.

The circulation line may comprise a gas circulation line circulating the purified NMP gas from the upstream side of the condenser to the recovery tower.

The circulation line may comprise a liquid circulation line circulating the condensed purified NMP from the downstream side of the condenser to the recovery tower.

The purified NMP may be introduced into the recovery tower through a gas circulation line and/or a liquid circulation line branched from the purified NMP line.

A waste NMP storage tank storing waste NMP containing high-boiling point impurities discharged from the bottom tower of the purification tower and a flow control valve for adjusting a flow rate of waste NMP introduced into the waste NMP storage tank may be provided.

The flow control valve may be an automatic control valve whose flow rate is automatically adjusted depending on the level of the waste NMP storage tank or the concentration of the waste NMP.

It is preferable that when the level of the purified NMP storage tank is equal to or higher than a preset level or the level of the recovered NMP storage tank is equal to or lower than a preset level upon normal operation of the purification device, a circulation mode is performed, in which normal operation of the purification device is maintained and high-purity NMP discharged from the purification device is circulated to the recovery device through the circulation line.

It is preferable that when the level of the purified NMP storage tank is lower than a preset level and the level of the recovered NMP storage tank is higher than a preset level upon normal operation of the purification device, a basic mode is performed, in which the recovered NMP is supplied to the purification device from the recovered NMP storage tank and the high-purity NMP purified in the purification device is discharged to the purified NMP storage tank.

The normal operation of the purification device can be called an operation in which purification is performed under optimal purification conditions in the purification device. A series of continuous and organic operations in which the recovered NMP to be purified is normally supplied to the purification device, the recovered NMP is normally purified, and the purified high-purity NMP is discharged can be called normal operation.

For the purified high-purity NMP discharged from the normal operation of the purification device, the basic mode is performed in which it is supplied to and stored in the purified NMP storage tank.

Circumstances, such as the case where the liquid level in the purified NMP storage tank reaches the upper limit, or the case where the coating process is temporarily stopped, thereby resulting in an excessive amount of purified NMP, may occur. In addition, the amount of purified NMP may also increase more than the amount of NMP required in the positive electrode mixing process.

Such excessive states of the purified NMP amount can be identified through the upper limit level inside the purified NMP storage tank.

In the positive electrode mixing process, the time (tact time) required to manufacture a main mixer slurry may be approximately 1 hour, and the times required to manufacture semifinished product (binder solution, pre-dispersion solution) mixers may be approximately 4 hours and 8 hours, respectively. Therefore, it can be said that the purified NMP is consumed, and then consumed again after a certain period has passed. Therefore, when the current purified NMP amount is excessive, the excessive state can be resolved after a certain period has passed.

It is problematic that there may always be a risk of operation stop of the purification tower until the excessive state of the purified NMP amount is resolved. That is, there is a problem that it becomes difficult to maintain normal operation.

Accordingly, in this example, for resolving the excessive state of the purified NMP amount and maintaining the normal operation of the purification tower, a circulation mode may be provided, in which the purified NMP due to normal operation of the purification tower is not supplied to the purified NMP storage tank, but is circulated and supplied to the recovery tower.

Meanwhile, circumstances, such as the case where the liquid level in the recovered NMP storage tank reaches the lower limit, or the case where the coating process is temporarily stopped, and thus the amount of recovered NMP is insufficient, may occur. That is, the amount of recovered NMP that should be supplied to the purification device may be insufficient.

Such insufficient states of the recovered NMP amount can be identified through the lower limit level inside the recovered NMP storage tank.

It is problematic that there may always be a risk of operation stop of the purification tower until the insufficient state of the NMP amount is resolved. That is, there is a problem that it becomes difficult to maintain normal operation.

Accordingly, in this example, for resolving the insufficient state of the recovered NMP amount and maintaining the normal operation of the purification tower, a circulation mode may be provided, in which the purified NMP due to the normal operation of the purification tower is not supplied to the purified NMP storage tank, but is circulated and supplied to the recovery tower.

Ultimately, in this example, to avoid the intentional temporary stop of the recovery device while avoiding the intentional temporary stop of the purification device, it is possible that the normal operation of the purification device and the recovery device can be maintained by circulating the purified NMP that the purification has been completed to the recovery device.

Through this, it is possible to prevent in advance the loss due to the restarting after the stop of the recovery device and the purification device, as well as the loss until the normal operation after the restarting. Furthermore, since the purified NMP in which the purification has been completed is partially used to recover the NMP, and then done again, it is possible to separate, particularly, the NMP peroxide more effectively during the recovery process and the purification process.

In order to achieve the above-described object, according to one example of the present invention, a control method for an NMP recovery purification system may be provided, which comprises: an NMP recovery step of introducing NMP contained in exhaust gas discharged from a secondary battery manufacturing process into a recovery tower, and condensing the introduced gas in the recovery tower to recover NMP; a recovered NMP storage step of storing the recovered NMP recovered and discharged in the recovery step in a recovered NMP storage tank; an NMP purification step of introducing the stored recovered NMP into a purification tower, and heating the recovered NMP introduced into the purification tower to purify the recovered NMP into high-purity NMP; a purified NMP storage step of storing the purified NMP purified and discharged in the purification step in a purified NMP storage tank; and a circulation step of bypassing the purified NMP purified and discharged in the purification step, and introducing it into the recovery tower.

The purified NMP storage step and the circulation step may be selectively performed.

The mode for storing the purified NMP can be called the basic mode, and the mode for circulating the purified NMP to the recovery device can be called the circulation mode. It is desirable that such basic and circulation modes are selectively performed during normal operation. When the purified NMP amount as currently required is excessive or the recovered NMP amount as required is insufficient, it can be said that the output amount is excessive or the input amount is insufficient from the perspective of the purification device. In this instance, it is preferable that the circulation mode is performed so that the temporary stop of the purification device is avoided and the normal operation of the purification device is maintained.

Through the NMP purification step, purified NMP gas may be discharged from the purification tower, and the purified NMP gas may be condensed through a condenser.

The circulation step may comprise a gas circulation step in which the purified NMP gas is introduced into the recovery tower, and a liquid circulation step in which the condensed purified NMP is introduced into the recovery tower.

To maintain normal performance of the NMP purification step in an insufficient state of the recovered NMP amount to be introduced into the purification tower, the circulation step may be performed.

The insufficient state of the recovered NMP amount may be determined based on the liquid level inside the recovered NMP storage tank. When the liquid level reaches the lower limit, the circulation mode may be performed.

To maintain normal performance of the NMP purification step in an excessive state of the purified NMP amount discharged from the purification tower, the circulation step may be performed.

The excessive state of the purified NMP amount may be determined based on the liquid level inside the purified NMP storage tank. When the liquid level reaches the upper limit, the circulation mode may be performed.

### Advantageous Effects

Through the present invention, it is an object to solve the problems of the conventional NMP recovery facility and the NMP purification facility.

Through one example of the present invention, it is possible to provide an NMP recovery purification system capable of maintaining normal operation of a purification tower despite a temporary stop condition of the purification tower by circulating NMP purified in the purification tower to a recovery tower, and a control method thereof.

Through one example of the present invention, it is possible to provide an NMP recovery purification system in which a circulation region is further expanded so that NMP purified in a purification tower is introduced into a recovery tower, and then introduced into the purification tower through the recovery tower again, instead of self-circulation in which it is introduced into the purification tower again, thereby capable of being in organic conjunction with the recovery tower and the purification tower, and securing safety and operational stability of the system, and a control method thereof.

Through one example of the present invention, it is possible to provide an NMP purification system capable of effectively reducing an impurity content in NMP, which is re-introduced into a purification tower, by further diluting the already-generated NMP peroxide in a recovery tower, and a control method thereof.

Through one example of the present invention, it is possible to provide an NMP recovery purification system capable of effectively preventing operation stop of a purification tower due to operation stop of a recovery tower by enabling normal operation of the recovery tower even under an operation stop condition of the recovery tower, and a control method thereof.

### Description of Drawings

Figure 1 is a simplified configuration diagram of an NMP recovery purification system according to one example of the present invention,
Figure 2 is a control flow diagram of an NMP recovery purification system according to one example of the present invention, and
Figure 3 illustrates a line configuration of an NMP recovery purification system according to one example of the present invention.

### Best Mode

Hereinafter, with reference to the attached drawings, an NMP recovery purification system according to one example of the present invention, and a control method thereof will be described in detail.

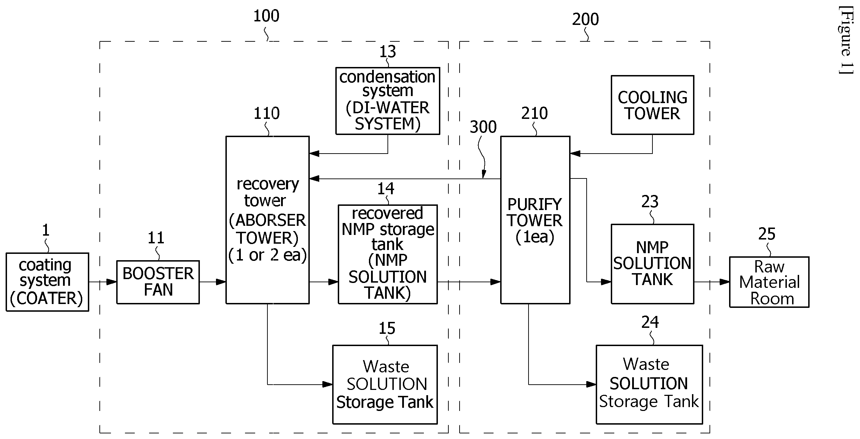

Figure 1 is a simplified configuration diagram of an NMP recovery purification system based on NMP.

As illustrated, a coating system (1, coater) in which electrode coating is performed, an NMP recovery system (100), and an NMP purification system (200) are substantially separated. Of course, each system may be connected through a pipe or line, and a recovery tower (110) and a purification tower (210), which form cores, may be located to be spaced apart from each other by a long distance at separate locations.

The exhaust gas discharged from the coating system (1) is introduced into the recovery tower (110) in a gaseous state through a booster fan (11). The recovery system (100) may comprise a recovery tower (110), a recovered NMP storage tank (14), and a waste NMP storage tank (15). The recovered NMP recovered through the operation of the recovery tower (110) is discharged from the recovery tower (110) and is introduced into the recovered NMP storage tank (14) and stored. Then, the waste NMP accumulated through the operation of the recovery tower (110) is introduced into the waste NMP storage tank (15) and stored. The waste NMP is an impurity including NMP peroxide, which may be a factor deteriorating the electrode performance, when it is included in a slurry.

The recovery tower (110) recovers NMP through various methods, where as one example, NMP may be recovered through condensation. To this end, a condensation system (13) for supplying pure water (DI-water, De-Ionized water inside the recovery tower, especially through the upper part of the recovery tower, may be provided to condendate NMP.

Specifically, the recovery system (100) comprising the recovery tower as a recovery device performs a function of recovering NMP used in the positive electrode material coater. NMP in a gaseous state is recovered, where an NMP liquid mixed with high-boiling point impurities and low-boiling point impurities (water) may be recovered into the recovery tank. In the recovery tower, NMP may be condensed and recovered using DI water, and the NMP may be recovered by various methods such as condensation, adsorption, and absorption. Therefore, the recovery tower may be implemented in a form having a bottom, a middle, and a top, and DI water may be supplied to the top. In addition, it may also be possible to implement a plurality of recovery towers connected to each other. As the high-temperature gaseous NMP rises, it is condensed and the NMP gathers at the bottom of the recovery tower, where moisture may be removed to increase the NMP concentration. The recovered NMP is stored in a recovered NMP tank.

Since the recovered NMP cannot be used as is in the positive electrode material mixing process, it can be purified and reused. The purification system (200) comprising the purification tower (210) as a purification device for purifying the recovered NMP purifies the recovered NMP and converts it into high-purity NMP, which is then reused in the positive electrode material mixing.

The purification tower (210) heats the recovered NPM to discharge high-purity gas NMP, where the discharged high-purity gas NMP is condensed and stored in the purified NMP tank (23). The purification tower (210) may be implemented in a form having a bottom, a middle, and a top, and the high-purity gas NMP may be discharged to the outside from the middle. Since the purified high-purity gas NMP is discharged from the middle of the purification tower, this can be called distillation in a side-cut manner.

The recovery system (100) and the purification system (200) are connected via the recovered NMP tank (14), but the recovery process and the purification process are performed individually. That is, the operation of the recovery tower (110) and the operation of the purification tower (210) may be individually controlled and performed.

In the recovery process and the purification process, waste NMP containing high boiling point impurities is separated and stored in each waste NMP storage tank (15, 24), and then took out to the outside, whereby a separate disposal process may be performed. By efficiently separating waste NMP in the recovery process and the purification process, high-purity NMP purification and reuse can be smoothly performed.

The purified NMP purified in the purification system (200) is stored in the purified NMP storage tank (23), and then supplied to a raw material room (25). The purified NMP supplied to the raw material room (25) may be used in the positive electrode material mixing process.

It is preferable that the electrode process of coating the positive electrode material after the positive electrode material mixing is performed continuously. To this end, NMP must be supplied appropriately. This means that the NMP purification process must be performed continuously and efficiently. In addition, in order that the purification process is performed continuously and efficiently, the recovery process of supplying the recovered NMP to the purification process must also be performed continuously and efficiently. That is, the recovery process and the purification process must be performed organically.

To perform the purification process continuously, the recovered NPM as the raw material must be supplied appropriately, and the purified NMP must be appropriately reused.

From the perspective of normal operation of the purification tower (210), when the purified NMP amount is excessive, normal operation of the purification tower is not easy, and even when the amount of recovered NMP as the raw material is insufficient, normal operation of the purification tower is not easy.

As one example, when the recovered NMP amount in the recovered NMP tank is insufficient (low level or lower limit level) or when the purified NMP amount in the purified NMP tank is excessive (high level or upper limit level), the purification process cannot be performed smoothly. When such a problem, i.e., an emergency of the purification tower, has occurred, conventionally, the problem has been temporarily solved by resupplying the NMP discharged after purification completion in the purification tower to the recovered NMP tank or adjusting the amount of discharged NMP. In other words, it has been intended to solve the problem in the purification tower itself.

However, since the purification process in the secondary battery manufacturing process must be performed organically with the electrode process or the recovery process, the emergency cause of the purification tower may not simply be the purification tower itself. Therefore, a fundamental and effective cause solution is necessary.

In this example, it is possible to provide a constitution, in which at least a portion of the high-purity NMP discharged after purification completion in the purification tower (210) is circulated to the recovery device (100), especially the recovery tower (110), through the circulation line (300) instead of discharging it to the purified NMP storage tank (23), and a control logic thereof. That is, by organically operating the recovery system (100) and the purification system (200), the emergency circumstances of the purification system (200) can be solved, and simultaneously, the recovery system (100), which is a cause thereof, can be normalized. In other words, the normal operation of the recovery system can be ensured or maintained as well as the normal operation of the purification system can be ensured or maintained.

In addition, in this example, it is possible to increase the separation efficiency of waste NMP while avoiding the operation stop of the recovery system (100) and the purification system (200) as much as possible. This is because as the already generated NMP peroxide is diluted in the recovery system (100) and discharged to the waste NMP tank (15) of the recovery system (100), the impurity content in the NMP re-introduced into the purification system (200) can be further reduced compared to the initial level.

The circulation line (300) may be connected to the bottom or middle of the recovery tower (110), and may also be connected to the top. At this time, it is preferable that the NMP re-supplied to the circulation line is liquid NMP. Therefore, it is connected, more preferably, to the top of the recovery tower. Of course, the NMP re-supplied to the circulation line may also be gaseous NMP. As one example, liquid NMP and gaseous NMP may also be selectively or simultaneously supplied to the recovery tower. At this time, the circulation line (300) may comprise multiple branch lines (311, 312, 313), and the respective branch lines may be connected to the recovery tower (110) at different locations.

Meanwhile, the recovery tower may be provided as a single tower or as multiple towers. As NMP flows from the recovery tower at the front to the recovery tower at the back, the concentration of the recovered NMP may increase. Therefore, it is preferable that the circulation line is connected to the recovery tower at the back.

When the recovery tower is provided as multiple towers, the NMP discharged from the first recovery tower may be re-introduced into the first recovery tower via the second recovery tower. Then, the recovered NMP may be finally discharged from the first recovery tower. In this case, the second recovery tower may recover NMP in a liquid state from NMP in a gaseous state and supply it to the specific recovery tower.

Multiple recovery towers with the same type may also be provided. That is, the NMP recovery capacity may be distributed to multiple recovery towers. In this case, the circulation lines extending from the single purification tower may be connected to the respective recovery towers.

When the purification tower (210) is normally operated, the purification process is continuously performed and the purified high-purity NMP is discharged. The mode in which the high-purity NMP is stored in the purified NMP storage tank may be referred to as the basic mode.

When the purification tower (210) is normally operated in the basic mode, the normal operation may be interrupted due to various causes. As one example, the cause may be a case where the high-purity purified NMP amount is excessive or the case where the amount of the recovered NMP, which is a raw material for purification, is insufficient.

According to the present example, upon normal operation of the purification device, when the level of the purified NMP storage tank is lower than the preset level and when the level of the recovered NMP storage tank is higher than the preset level, it is preferable that the basic mode in which the recovered NMP is supplied to the purification device from the recovered NMP storage tank and the high-purity NMP purified in the purification device is discharged to the purified NMP storage tank is performed.

According to the present example, upon normal operation of the purification device, when the level of the purified NMP storage tank is equal to or higher than the preset level or when the level of the recovered NMP storage tank is equal to or lower than the preset level, it is preferable that the circulation mode in which normal operation of the purification device is maintained and the high-purity NMP discharged from the purification device is circulated to the recovery device through the circulation line is performed.

The normal operation of the purification device can be said to be an operation in which purification is performed under optimal purification conditions in the purification device. A series of continuous and organic operations in which the recovered NMP to be purified is normally supplied to the purification device, the recovered NMP is normally purified, and the purified high-purity NMP is discharged can be said to be normal operation.

The basic mode is performed in which the purified high-purity NMP discharged in the normal operation of the purification device is supplied to the purified NMP storage tank and stored.

Circumstances, such as the case where the liquid level in the purified NMP storage tank reaches the upper limit, or the case where the coating process is temporarily stopped, thereby resulting in an excessive amount of purified NMP, may occur. In addition, the amount of purified NMP may also increase more than the amount of NMP required in the positive electrode mixing process.

Such excessive states of the purified NMP amount can be identified through the upper limit level inside the purified NMP storage tank.

In the positive electrode mixing process, the time (tact time) required to manufacture a main mixer slurry may be approximately 1 hour, and the times required to manufacture semifinished product (binder solution, pre-dispersion solution) mixers may be approximately 4 hours and 8 hours, respectively. Therefore, it can be said that the purified NMP is consumed, and then consumed again after a certain period has passed. Therefore, when the current purified NMP amount is excessive, the excessive state can be resolved after a certain period has passed.

It is problematic that there may always be a risk of operation stop of the purification tower until the excessive state of the purified NMP amount is resolved. That is, there is a problem that it becomes difficult to maintain normal operation.

Accordingly, in this example, for resolving the excessive state of the purified NMP amount and maintaining the normal operation of the purification tower, a circulation mode may be provided, in which the purified NMP due to normal operation of the purification tower is not supplied to the purified NMP storage tank, but is circulated and supplied to the recovery tower.

Meanwhile, circumstances, such as the case where the liquid level in the recovered NMP storage tank reaches the lower limit, or the case where the coating process is temporarily stopped, and thus the amount of recovered NMP is insufficient, may occur. That is, the amount of recovered NMP that should be supplied to the purification device may be insufficient.

Such insufficient states of the recovered NMP amount can be identified through the lower limit level inside the recovered NMP storage tank.

It is problematic that there may always be a risk of operation stop of the purification tower until the insufficient state of the NMP amount is resolved. That is, there is a problem that it becomes difficult to maintain normal operation.

Accordingly, in this example, for resolving the insufficient state of the recovered NMP amount and maintaining the normal operation of the purification tower, a circulation mode may be provided, in which the purified NMP due to the normal operation of the purification tower is not supplied to the purified NMP storage tank, but is circulated and supplied to the recovery tower.

Ultimately, in this example, to avoid the intentional temporary stop of the recovery device while avoiding the intentional temporary stop of the purification device, it is possible that the normal operation of the purification device and the recovery device can be maintained by circulating the purified NMP that the purification has been completed to the recovery device.

Through this, it is possible to prevent in advance the loss due to the restarting after the stop of the recovery device and the purification device, as well as the loss until the normal operation after the restarting. Furthermore, since the purified NMP in which the purification has been completed is partially used to recover the NMP, and then done again, it is possible to separate, particularly, the NMP peroxide more effectively during the recovery process and the purification process.

The low level of the recovered NMP storage tank and the high level of the purified NMP storage tank may be controlled by being separated into at least two stages. As one example, a first low level and a first high level for performing the circulation mode may be sensed. While performing the circulation mode, if a level equal to or higher than the first low level is sensed and a level equal to or lower than the first high level is sensed, the circulation mode may be stopped and the normal purification process may be performed. Of course, the normal recovery process may also be performed. While performing the circulation mode, when a second low level lower than the first low level is sensed and a second high level higher than the first high level is sensed, follow-up measures such as increasing an NMP circulation amount may be performed. If necessary, the purification process may also be stopped. However, when the purification process is stopped, a stabilization time upon restarting, and a yield loss are expected, so that it is preferable to avoid stopping the purification process as much as possible.

Meanwhile, the recovery tower and the purification tower are divided into the top, middle, and bottom, which are distinguished through their positions in the entire tower, where it is also possible that each stage is operated as a single recovery structure or purification structure, or as multiple recovery structures or purification structures.

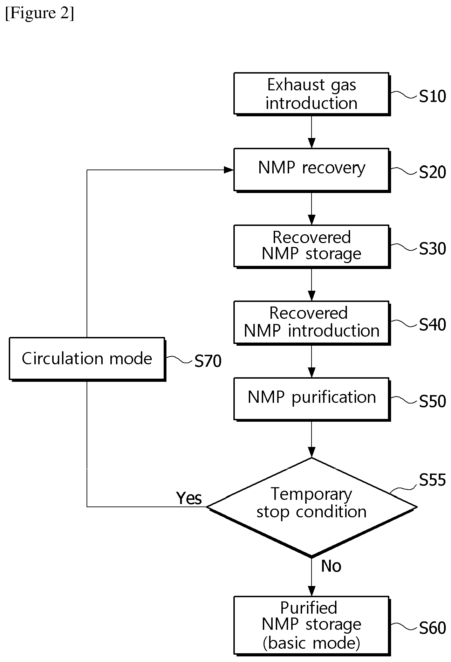

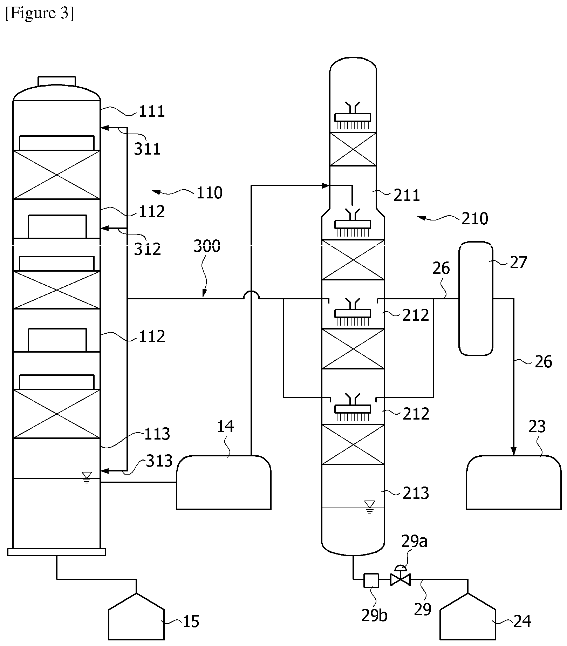

Hereinafter, the control logic and main constitutions of the NMP recovery purification system will be described in detail with reference to Figures 2 and 3.

Exhaust gas discharged from the coating process is introduced into the recovery tower (110) through a blower, and the like (S10). The recovery tower (110) may be composed of a top layer (111), a middle layer (112), and a bottom layer (113).

The exhaust gas introduced into the recovery tower in a high-temperature state is condensed by condensation water while rising, and thus collected in the bottom layer (113). DI water for condensation may be supplied to the inside of the upper layer in the recovery tower (110), and the NMP condensate collected in the bottom layer may be circulated and introduced to the inside of the top layer, so that the NMP in the exhaust gas may be separated.

The NMP may be recovered by removing water from the NMP condensate collected in the bottom layer. Approximately 50 to 85 wt% of NMP may be recovered through the recovery process.

The NMP recovered in the recovery tower (110) is introduced into the recovered NMP storage tank (14) and stored.

The stored recovered NMP becomes a raw material for NMP purification, is purified into high-purity NMP through the purification process, and is reused in a subsequent coating process.

The purification device may comprise a purification tower (210), and the purification tower (210) may also be formed into multiple layers. For example, it may be formed into a top layer (211), a middle layer (212), and a bottom layer (213), where the middle layer may be formed by multiple layers.

The recovered NMP introduced into the purification tower (210) may be heated inside the purification tower, and purified through a distillation process, and as the purified NMP is discharged from the middle layer (212), a side-cut distillation method may be applied.

The high-purity purified NMP discharged from the purification tower (210) is stored in the purified NMP storage tank (23) through the purified NMP line (26). That is, upon normal operation, the purification tower (210) may continuously produce high-purity purified NMP, and the high-purity purified NMP may be stored in the basic mode.

In this example, a waste NMP storage tank (24) for storing waste NMP containing high-boiling point impurities discharged from the bottom tower of the purification tower and a flow control valve (29a) for adjusting a flow rate of waste NMP flowing into the waste NMP storage tank may be included. The flow control valve may be provided on the waste NMP line (29).

The flow control valve (29a) may be provided to control the flow rate of the waste NMP to be discharged by automatically adjusting an opening degree. It is preferable that the flow control is performed based on the results of analyzing the components and contents of impurities in the waste NMP. An analytical instrument (29b) capable of analyzing the components and contents of impurities in waste NMP flowing into the waste NMP storage tank in real time in-line is provided, and the flow control may be performed based on the results analyzed by the analytical instrument.

The analytical instrument (29b) may be provided to automatically adjust the opening degree of the flow control valve (29a) by monitoring the waste NMP concentration. That is, by monitoring the waste NMP concentration to quantitatively adjust an opening rate, it is possible to secure a freedom degree capable of implementing advancement of the NMP recovery and purification system from the perspective of control, yield improvement, and the like.

This monitoring of the waste NMP concentration may be performed online or offline, and when the waste NMP concentration is low, the opening rate may be reduced to increase the NMP recovery rate, and when the concentration is high, the opening rate may be increased to increase the recovery efficiency. The analytical instrument (29b) may be a gas chromatography mass spectrometer such as a GC-MS analyzer.

As described above, the waste NMP may be separated in both the recovery tower (110) and the purification tower (210). That is, it is preferable that NMP peroxide is separated as much as possible, and ultimately not included in the high-purity purified NMP.

According to the present example, high-purity purified NMP may be again introduced into the recovery tower (110) under certain conditions. That is, upon normal operation of the purification tower (210), it may be introduced into the recovery tower (110) under certain conditions, and used once again in the recovery process.

Therefore, waste NMP may be filtered out again in the recovery tower, which means that the NMP peroxide introduced into the purification tower (210) is further reduced. As a result, the purity of the high-purity purified NMP can be further increased.

The purified NMP circulation line (300) may be connected to the purification tower (210) in the same form as the purified NMP line (26). That is, the purified NMP circulation line (300) and the purified NMP line (26) may be selectively opened.

In addition, the purified NMP circulation line (300) may be provided by branching from the purified NMP line (26). That is, the purified NMP discharged through the purified NMP line may be continuously introduced into the storage tank (23) or introduced into the recovery tower (110) along the circulation line (300).

The purified NMP line and circulation line may be provided with valves for controlling the opening and closing of the flow path, respectively, and, if necessary, a flow control valve.

Meanwhile, the NMP discharged through the purified NMP line (26) may be in a gaseous state, and may be converted into a liquid state through the condenser (27). This means that the gaseous NMP can be branched and circulated from the purified NMP line (26) or the liquid NMP can be branched and circulated therefrom.

Therefore, in the circulation mode, the high-purity purified NMP in a liquid or gaseous state may be circulated to the recovery tower (110) as needed. At this time, it may also be possible to selectively circulate the NMP to the top layer, middle layer, or bottom layer of the recovery tower (110). As one example, liquid NMP can be introduced into the bottom layer of the recovery tower and gaseous NMP can be introduced into the top layer of the recovery tower. Of course, the opposite can also be done.

In other words, in the circulation mode, the normal operation of the recovery tower can be continuously maintained, and the purified NMP introduced into the recovery tower can be selected as gas or liquid by considering the current conditions inside the recovery tower, such as the concentration of the recovered NMP, temperature, and pressure. Of course, the location to which the purified NMP is introduced can also be selected.

Therefore, according to the present example, the normal operation of the purification device can be continuously maintained, and the normal operation of the recovery tower can also be continuously maintained. In addition, since the separation of waste NMP can be additionally performed in the circulation mode, the purity of the purified NMP can be further increased.

As shown in Figure 2, from the perspective of the recovery tower, exhaust gas introduction (S10), NMP recovery (S20), and recovered NMP storage (S30) are organically and continuously performed upon normal operation. From the perspective of the purification tower, introduction of recovered NMP into the purification tower (S40), NMP purification (S50), and purified NMP storage (S60) are organically and continuously performed upon normal operation. That is, the basic mode is performed.

However, when a temporary stop condition of the purification tower occurs (S55), the circulation mode (S70) can be performed without temporarily stopping the purification tower. One of the temporary stop conditions of the purification tower may also be a temporary stop condition of the recovery tower. That is, by performing the circulation mode, the temporary stop condition of the purification tower and the temporary stop condition of the recovery tower are resolved, so that normal operation of both can be continued.

The temporary stop conditions can be various, and various speed changes or sensing values for determining these can be preset. As one example, it is possible to determine whether the purified NMP storage amount is excessive based on the liquid level of the purified NMP storage tank, and it is also possible to determine whether the recovered NMP storage amount is insufficient based on the liquid level of the recovered NMP storage tank.

Such a circulation mode does not circulate NMP through the purification tower or the purification system itself, but extends to the recovery tower region and circulates it. Therefore, it is possible to stably configure and operate the control logic interlocking up to the use, recovery, and purification of NMP.

### Industrial Applicability

It is described in the Detailed Description of Invention.

## Claims

1. An NMP recovery purification system, comprising:
a recovery device operating to recover NMP contained in exhaust gas discharged from a secondary battery process;
a recovered NMP storage tank storing the recovered NMP recovered and discharged from the recovery device;
a purification device operating to purify the recovered NMP supplied from the recovered NMP storage tank into high-purity NMP;
a purified NMP storage tank storing the purified NMP purified and discharged from the purification device; and
a circulation line circulating the purified NMP discharged from the purification device to the recovery device.

2. The NMP recovery purification system according to claim 1, **characterized in that**
the recovery device comprises a recovery tower, and the purification device comprises a purification tower.

3. The NMP recovery purification system according to claim 2, **characterized in that**
the purification tower has a top tower for separating high-concentration NMP and low-boiling-point impurities, a bottom tower for separating high-purity NMP and high-boiling-point impurities, and a middle tower for discharging high-purity purified NMP gas between the top tower and the bottom tower.

4. The NMP recovery purification system according to claim 3, **characterized in that**
the purification device comprises a condenser for condensing the high-purity purified NMP gas discharged from the middle tower to supply it to the purified NMP storage tank.

5. The NMP recovery purification system according to claim 4, **characterized in that**
the circulation line is provided to connect the purification tower and the recovery tower.

6. The NMP recovery purification system according to claim 5, **characterized in that**
the circulation line comprises a gas circulation line circulating the purified NMP gas from the upstream side of the condenser to the recovery tower.

7. The NMP recovery purification system according to claim 5, **characterized in that**
the circulation line comprises a liquid circulation line circulating the condensed purified NMP from the downstream side of the condenser to the recovery tower.

8. The NMP recovery purification system according to claim 4, **characterized in that**
a purified NMP line, through which the purified NMP discharged from the purification tower is transferred to the purified NMP storage tank, is included, and
the circulation line is branched from the purified NMP line and connected to the recovery tower.

9. The NMP recovery purification system according to claim 3, **characterized by** comprising
a waste NMP storage tank storing waste NMP containing high-boiling point impurities discharged from the bottom tower of the purification tower and a flow control valve for adjusting a flow rate of waste NMP introduced into the waste NMP storage tank.

10. The NMP recovery purification system according to claim 9, **characterized in that**
the flow control valve is an automatic control valve whose flow rate is automatically adjusted depending on the level of the waste NMP storage tank or the concentration of the waste NMP.

11. The NMP recovery purification system according to any one of claims 1 to 10, **characterized in that**
when the level of the purified NMP storage tank is equal to or higher than a preset level or the level of the recovered NMP storage tank is equal to or lower than a preset level upon normal operation of the purification device, a circulation mode is performed, in which normal operation of the purification device is maintained and high-purity NMP discharged from the purification device is circulated to the recovery device through the circulation line.

12. The NMP recovery purification system according to claim 11, **characterized in that**
when the level of the purified NMP storage tank is lower than a preset level and the level of the recovered NMP storage tank is higher than a preset level upon normal operation of the purification device, a basic mode is performed, in which the recovered NMP is supplied to the purification device from the recovered NMP storage tank and the high-purity NMP purified in the purification device is discharged to the purified NMP storage tank.

13. A control method for an NMP recovery purification system comprising:
an NMP recovery step of introducing NMP contained in exhaust gas discharged from a secondary battery manufacturing process into a recovery tower, and condensing the introduced gas in the recovery tower to recover NMP;
a recovered NMP storage step of storing the recovered NMP recovered and discharged in the recovery step in a recovered NMP storage tank;
an NMP purification step of introducing the stored recovered NMP into a purification tower, and heating the recovered NMP introduced into the purification tower to purify the recovered NMP into high-purity NMP;
a purified NMP storage step of storing the purified NMP purified and discharged in the purification step in a purified NMP storage tank; and
a circulation step of bypassing the purified NMP purified and discharged in the purification step, and introducing it into the recovery tower.

14. The control method for an NMP recovery purification system according to claim 13, **characterized in that**
the purified NMP storage step and the circulation step are selectively performed.

15. The control method for an NMP recovery purification system according to claim 13, **characterized in that**
through the NMP purification step, purified NMP gas is discharged from the purification tower, and the purified NMP gas is condensed through a condenser.

16. The control method for an NMP recovery purification system according to claim 15, **characterized in that**
the circulation step comprises a gas circulation step in which the purified NMP gas is introduced into the recovery tower, and a liquid circulation step in which the condensed purified NMP is introduced into the recovery tower.

17. The control method for an NMP recovery purification system according to any one of claims 13 to 16, **characterized in that**
to maintain normal performance of the NMP purification step in an insufficient state of the recovered NMP amount to be introduced into the purification tower, the circulation step is performed.

18. The control method for an NMP recovery purification system according to claim 17, **characterized in that**
the insufficient state of the recovered NMP amount is determined based on the liquid level inside the recovered NMP storage tank.

19. The control method for an NMP recovery purification system according to any one of claims 13 to 16, **characterized in that**
to maintain normal performance of the NMP purification step in an excessive state of the purified NMP amount discharged from the purification tower, the circulation step is performed.

20. The control method for an NMP recovery purification system according to claim 19, **characterized in that**
the excessive state of the purified NMP amount is determined based on the liquid level inside the purified NMP storage tank.
